# EUROPEAN PATENT APPLICATION

(11) **EP 4 559 907 A1**
(43) Date of publication of application: **28.05.2025**
(21) Application number: 23845417.7
(22) Date of filing: 20.07.2023
(51) Int. Cl.: C07D 403/04, A61K 31/506, A61P 35/00

(54) **CRYSTAL FORM OF PIPERAZINE AMIDE DERIVATIVE, PREPARATION METHOD THEREFOR AND USE THEREOF**

(30) Priority: 23.07.2022 CN 202210872564
(71) Applicant: Zhejiang Hisun Pharmaceutical Co., Ltd., Taizhou, Zhejiang 318000 (CN)
(72) Inventor: CHEN, Lianwei, Taizhou, Zhejiang 318000 (CN); ZHAO, Meiyu, Taizhou, Zhejiang 318000 (CN); LI, Na, Taizhou, Zhejiang 318000 (CN); SHI, Zhenjuan, Taizhou, Zhejiang 318000 (CN)
(74) Representative: Boult Wade Tennant LLP
(86) International application number: PCT/CN2023/108281
(87) International publication number: WO 2024/022206

(57) **Abstract**

The present disclosure relates to the field of chemical pharmaceuticals, relates to a crystal form of a piperazine amide derivative, a preparation method therefor and a use thereof, specifically relates to crystal forms A, B, C, D, and E of a compound of formula (I), a preparation method therefor, and a pharmaceutical composition and use thereof, and solves the problems of small granularity, poor fluidity, poor stability and the like of the compound of formula (I) present in an amorphous form. The crystal form prepared by the present disclosure and the pharmaceutical composition thereof can be used for preparing a drug for treating diseases such as cancer.

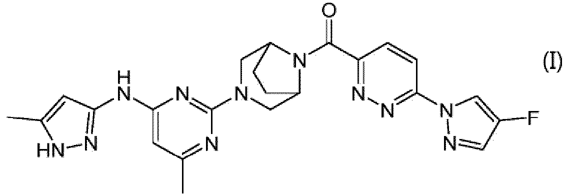

## Description

### FIELD

The present disclosure relates to the technical field of chemical pharmaceutics, particularly relates to novel crystal forms A, B, C, D, and E of a piperazine amide derivative (6-(4-fluoro-1H-pyrazol-1-yl)pyridazin-3-yl)(3-(4-methyl-6-((5-methyl-1H-pyrazol-3-yl)amino)p yrimidin-2-yl)-3,8-diazabicyclo[3.2.1]octan-8-yl)methanone, and a preparation method therefor, further particularly relates to a pharmaceutical composition comprising same and use thereof.

### BACKGROUND

Rearranged during transfection gene (RET gene) is a proto-oncogene that encodes a tyrosine kinase receptor in the human body and regulates cell reproduction and survival. This gene is activated by the interaction with glial cell-derived neurotrophic factor family receptors and α receptors of the family to form a dimer. Through phosphorylation, it regulates signal pathways, exercises the functions of signaling and regulating life activities. Abnormal expression of RET gene is associated with a variety of cancer diseases. The gene fused with other genes through chromosomal rearrangement or experiencing site-directed mutation can be continuously in activated state independently of ligands, leading to abnormal signaling pathways, thereby causing excessive cell proliferation and cancer.

In recent years, more and more evidences have shown that the fusion and mutation of RET gene are the driving force of some cancers, and RET gene does not coincide with other driver genes, presenting significant specificity. RET gene fusion is common in papillary thyroid cancer and non-small cell lung cancer. For example, 30% of sporadic papillary thyroid cancer, 70% of radiation-induced papillary thyroid cancer, and about 2% of non-small cell lung cancer are driven by RET gene fusion. RET gene mutation is common in medullary thyroid cancer. For example, more than 50% of medullary thyroid cancer and almost all congenital medullary cancer and multiple endocrine adenomatosis are caused by site-directed mutations in RET gene.

Current treatment methods mainly use multi-target kinase inhibitors with RET kinase inhibitory activity to treat cancer patients with RET gene fusion or mutation. However, under these conditions, due to off-target effects and drug toxicity, the dose of the drug is insufficient to achieve a level sufficient to inhibit abnormal expression of RET gene. In addition, in the process of cancer treatment, cancer cells will develop drug resistance through mutations. Once drug resistance occurs, the patient's treatment options will become very limited. Therefore, there is a great need for a selective RET kinase inhibitor to treat patients with RET gene fusion or mutation.

WO2020/207419A1 discloses a piperazine amide derivative having the structure represented by Formula I, with the molecular formula of C₂₃H₂₄FN₁₁O and the chemical name of (6-(4-fluoro-1H-pyrazol-1-yl)pyridazin-3-yl)(3-(4-methyl-6-((5-methyl-1H-pyrazol-3-yl)amino)p yrimidin-2-yl)-3,8-diazabicyclo[3.2.1]octan-8-yl)methanone.WO2020/207419A1 also discloses a preparation method for the compound represented by formula I and the use thereof as a RET kinase inhibitor, indicating that the compound has a good effect of inhibiting RET kinase.

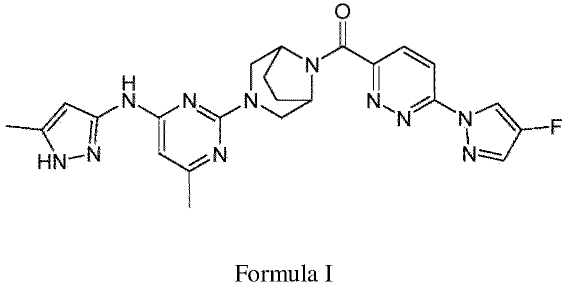

However, the above compound represented by Formula I disclosed in WO2020/207419A1 prepared by the preparation method thereof is a solid in an amorphous form, and its properties such as stability, granularity, fluidity, and hygroscopicity are still not satisfactory. In particular, the amorphous particles disclosed in WO2020/207419A1 have small granularity and poor fluidity, and are not suitable for the forming process of solid preparations (such as tablets, capsules or granules).

Currently, in the prior art, there is neither a form of the compound represented by Formula I that is suitable for preparation formation and has satisfactory stability, nor report on the crystal form of the compound represented by Formula I. The inventors have obtained the crystal forms of the compound represented by Formula I through a large number of experiments.

### SUMMARY

In view of the above-mentioned problems in the prior art, the present disclosure provides crystal forms of a compound represented by Formula I (6-(4-fluoro-1H-pyrazol-1-yl)pyridazin-3-yl)(3-(4-methyl-6-((5-methyl-1H-pyrazol-3-yl)amino)p yrimidin-2-yl)-3,8-diazabicyclo[3.2.1]octan-8-yl)methanone with the following structure and a preparation method thereof.

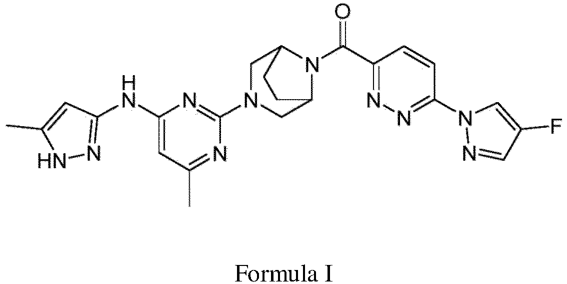

In a first aspect, the present disclosure provides a crystal form A of the compound represented by Formula I (hereinafter referred to as "crystal form A").

The crystal form A has an X-ray powder diffraction pattern with Cu-Kα radiation comprising characteristic peaks at 2θ of 8.5±0.2°, 11.4±0.2°, 14.4±0.2°, 15.7±0.2°, 16.7±0.2°, 17.8±0.2°, 25.1±0.2° and 28.1±0.2° in degrees.

Preferably, the crystal form A has an X-ray powder diffraction pattern with Cu-Kα radiation comprising characteristic peaks at 2θ of 7.0±0.2°, 8.5±0.2°, 11.4±0.2°, 13.1±0.2°, 14.4±0.2°, 15.7±0.2°, 16.7±0.2°, 17.8±0.2°, 20.2±0.2°, 21.2±0.2°, 22.0±0.2°, 23.1±0.2°, 24.3±0.2°, 25.1±0.2°, 26.2±0.2° and 28.1±0.2° in degrees.

More preferably, the X-ray powder diffraction pattern with Cu-Kα radiation of the crystal form A shows characteristic peaks at 2θ of the following positions in degrees and relative intensities, and the details are shown in Table 1.

**Table 1**

| number of peak | 2θ (°) | d (Å) | relative intensity (%) |
|---|---|---|---|
| 1 | 7.0 | 12.5 | 6.1 |
| 2 | 8.5 | 10.4 | 44.8 |
| 3 | 11.4 | 7.8 | 56.1 |
| 4 | 13.1 | 6.8 | 5 |
| 5 | 14.0 | 6.3 | 15.2 |
| 6 | 14.4 | 6.2 | 31.1 |
| 7 | 14.8 | 6.0 | 8 |
| 8 | 15.7 | 5.6 | 31.1 |
| 9 | 16.0 | 5.5 | 7.8 |
| 10 | 16.7 | 5.3 | 54.9 |
| 11 | 17.2 | 5.2 | 11.5 |
| 12 | 17.8 | 5.0 | 100 |
| 13 | 20.2 | 4.4 | 15.5 |
| 14 | 20.6 | 4.3 | 10.1 |
| 15 | 21.2 | 4.2 | 7.2 |
| 16 | 22.0 | 4.0 | 8.4 |
| 17 | 22.3 | 4.0 | 4.2 |
| 18 | 23.1 | 3.8 | 9.5 |
| 19 | 23.4 | 3.8 | 2.9 |
| 20 | 24.3 | 3.7 | 16.2 |
| 21 | 25.1 | 3.5 | 22.6 |
| 22 | 26.2 | 3.4 | 27.1 |
| 23 | 26.4 | 3.4 | 19.7 |
| 24 | 27.5 | 3.2 | 4.4 |
| 25 | 28.1 | 3.2 | 26.9 |
| 26 | 28.7 | 3.1 | 5.5 |
| 27 | 29.4 | 3.0 | 4.7 |
| 28 | 31.4 | 2.8 | 1.1 |
| 29 | 31.9 | 2.8 | 1.1 |
| 30 | 32.6 | 2.7 | 4.8 |
| 31 | 33.5 | 2.7 | 1 |
| 32 | 34.5 | 2.6 | 1.2 |
| 33 | 35.0 | 2.6 | 0.8 |
| 34 | 35.5 | 2.5 | 0.9 |
| 35 | 37.5 | 2.4 | 0.9 |
| 36 | 39.6 | 2.3 | 3.1 |

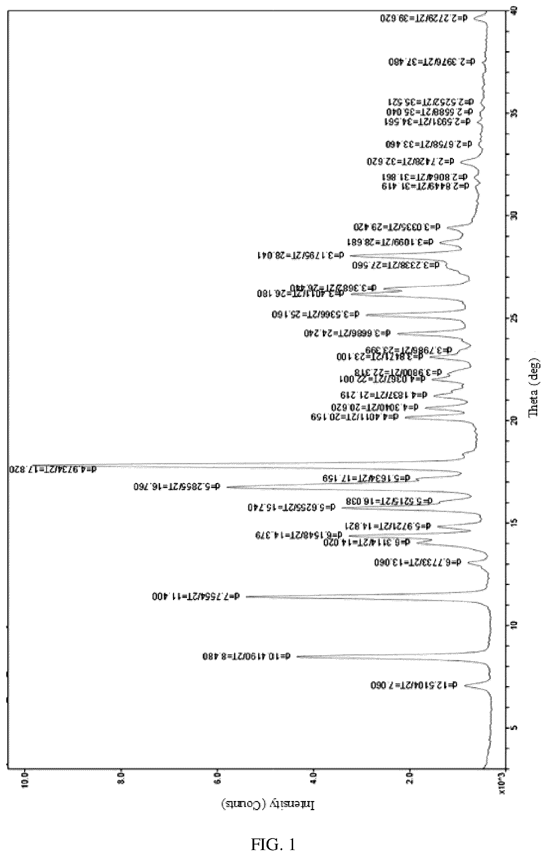

More preferably, the crystal form A has an X-ray powder diffraction pattern substantially as shown in FIG. 1.

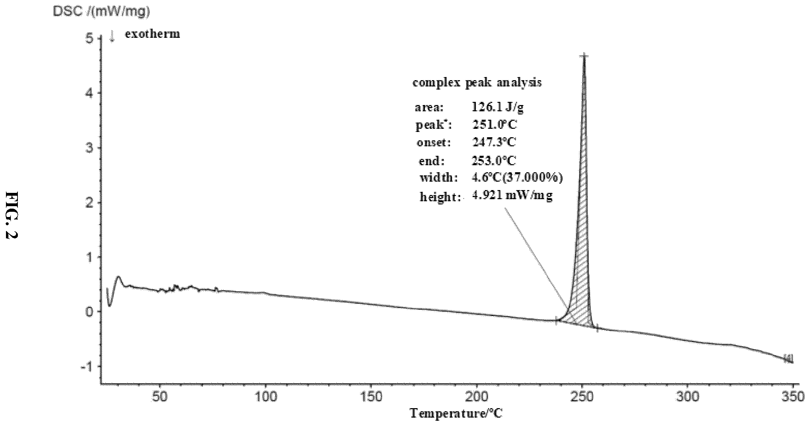

Further, the crystal form A has a differential scanning calorimetry (DSC) trace comprising an endothermic peak at 247°C to 253°C, and the DSC trace is substantially as shown in FIG. 2.

Correspondingly, the present disclosure provides a method for preparing the crystal form A, comprising
adding an amorphous form of a compound represented by Formula I to a solvent to obtain a suspension, stirring the suspension at 5°C to 50°C for 1 to 7 days, separating a solid, and drying the solid under vacuum to obtain the crystal form A, and the solvent is selected from the group consisting of methanol, ethanol, n-propanol, isopropanol, acetone, methyl isobutyl ketone, 2-butanone, and a combination thereof.

Preferably, a weight-volume ratio of the compound represented by Formula I to the solvent is 1:10-1:50 in a unit of g/mL.

Preferably, the drying under vacuum is carried out at a temperature of 20°C to 60°C for 8 to 24 hours.

In another aspect, the present disclosure provides a pharmaceutical composition comprising a therapeutically effective amount of the crystal form A as an active ingredient. Preferably, in the pharmaceutical composition, the crystal form A can be mixed with one or more pharmaceutically acceptable solid or liquid diluents and/or excipients to prepare into a galenic preparation.

In a still another aspect, the present disclosure provides use of crystal form A or a pharmaceutical composition thereof in the manufacture of a medicament for treating a disease related to RET gene fusion or mutation, wherein the disease is preferably a cancer, and the cancer is preferably breast cancer, non-small cell lung cancer, or rectal cancer.

In a still another aspect, the present disclosure provides use of the crystal form A or a pharmaceutical composition thereof in the manufacture of a RET kinase inhibitor.

In addition, the present disclosure further provides a method for the treatment of a disease related to a RET gene fusion or mutation, and the method comprises administering a therapeutically effective amount of the crystal form A to a subject in need thereof, wherein the disease is preferably a cancer, wherein the cancer is preferably breast cancer, non-small cell lung cancer, or rectal cancer.

The present disclosure further provides a method for inhibiting RET kinase, and the method comprises contacting the crystal form A or a pharmaceutical composition thereof with RET kinase.

In another aspect, the present disclosure provides a crystal form B of the compound represented by Formula I (hereinafter referred to as "crystal form B").

The crystal form B has an X-ray powder diffraction pattern with Cu-Kα radiation comprising characteristic peaks at 2θ of 5.9±0.2°, 11.8±0.2°, 13.4±0.2°, 17.7±0.2°, 23.6±0.2° and 26.9±0.2° in degrees.

Preferably, the crystal form B has an X-ray powder diffraction pattern with Cu-Kα radiation comprising characteristic peaks at 2θ of 5.9±0.2°, 9.0±0.2°, 9.9±0.2°, 11.8±0.2°, 13.4±0.2°, 17.7±0.2°, 21.9±0.2°, 23.6±0.2°, 24.5±0.2°, 26.9±0.2° and 29.9±0.2° in degrees.

More preferably, the X-ray powder diffraction pattern with Cu-Kα radiation of the crystal form B shows characteristic peaks at 2θ of the following positions in degrees and relative intensities, and the details are shown in Table 2.

**Table 2**

| number of peak | 2θ (°) | d (Å) | relative intensity (%) |
|---|---|---|---|
| 1 | 5.9 | 15.0 | 100.0 |
| 2 | 9.0 | 9.8 | 3.6 |
| 3 | 9.9 | 8.9 | 6.6 |
| 4 | 11.8 | 7.5 | 14.0 |
| 5 | 13.4 | 6.6 | 21.5 |
| 6 | 14.8 | 6.0 | 6.1 |
| 7 | 15.2 | 5.8 | 6.5 |
| 8 | 16.0 | 5.5 | 8.1 |
| 9 | 16.5 | 5.4 | 6.5 |
| 10 | 17.7 | 5.0 | 16.5 |
| 11 | 18.5 | 4.8 | 7.5 |
| 12 | 19.1 | 4.6 | 6.3 |
| 13 | 19.6 | 4.5 | 4.8 |
| 14 | 20.9 | 4.2 | 5.0 |
| 15 | 21.9 | 4.1 | 7.7 |
| 16 | 23.2 | 3.8 | 11.8 |
| 17 | 23.6 | 3.8 | 20.3 |
| 18 | 24.5 | 3.6 | 7.7 |
| 19 | 26.9 | 3.3 | 71.1 |
| 20 | 27.7 | 3.2 | 19.2 |
| 21 | 29.9 | 3.0 | 5.6 |

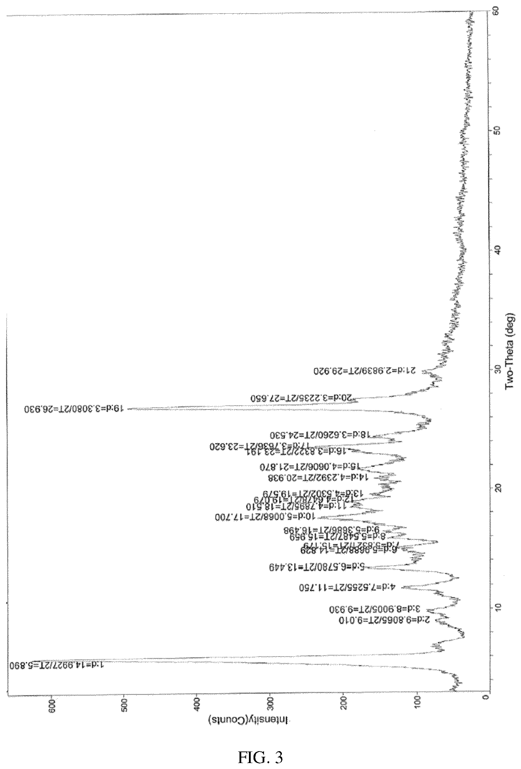

More preferably, the crystal form B has an X-ray powder diffraction pattern substantially as shown in FIG. 3.

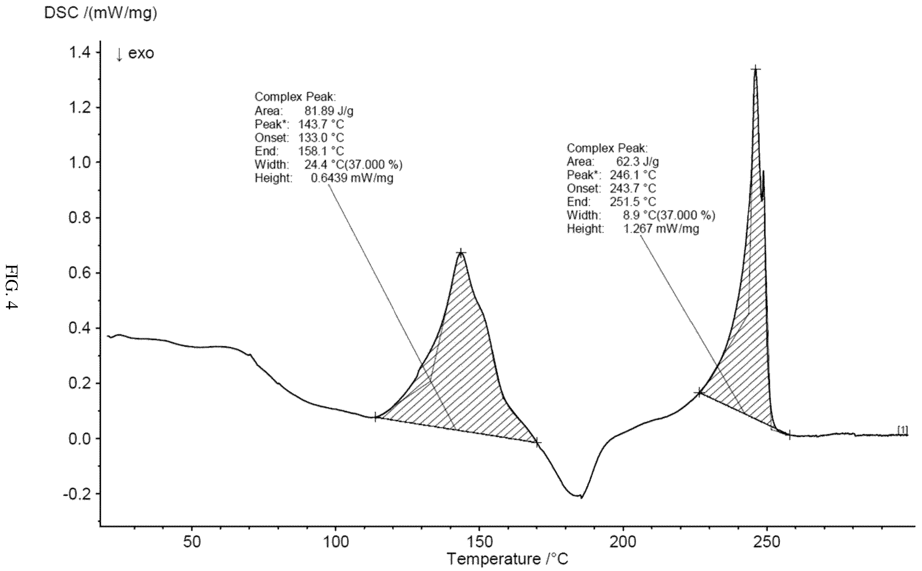

Further, the crystal form B has a DSC trace comprising an endothermic peak at 133°C to 158°C, and 243°C to 251°C, and the DSC trace is substantially as shown in FIG. 4.

Correspondingly, the present disclosure provides a method for preparing the crystal form B, comprising

adding an amorphous form of a compound represented by Formula I to dichloromethane to obtain a suspension, stirring the suspension at 5°C to 35°C for 1 to 7 days, separating a solid, and drying the solid under vacuum to obtain the crystal form B.

Preferably, a weight-volume ratio of the compound represented by Formula I to dichloromethane is 1:10-1:50 in a unit of g/mL.

Preferably, the drying under vacuum is carried out at a temperature of 20°C to 60°C for 8 to 24 hours.

In another aspect, the present disclosure provides a pharmaceutical composition comprising a therapeutically effective amount of the crystal form B as an active ingredient. Preferably, in the pharmaceutical composition, the crystal form B can be mixed with one or more pharmaceutically acceptable solid or liquid diluents and/or excipients to prepare into a galenic preparation.

In a still another aspect, the present disclosure provides use of crystal form B or a pharmaceutical composition thereof in the manufacture of a medicament for treating a disease related to RET gene fusion or mutation, wherein the disease is preferably a cancer, and the cancer is preferably breast cancer, non-small cell lung cancer, or rectal cancer.

In a still another aspect, the present disclosure provides use of the crystal form B or a pharmaceutical composition thereof in the manufacture of a RET kinase inhibitor.

In addition, the present disclosure further provides a method for the treatment of a disease related to a RET gene fusion or mutation, and the method comprises administering a therapeutically effective amount of the crystal form B to a subject in need thereof, wherein the disease is preferably a cancer, wherein the cancer is preferably breast cancer, non-small cell lung cancer, or rectal cancer.

The present disclosure further provides a method for inhibiting RET kinase, and the method comprises contacting the crystal form B or a pharmaceutical composition thereof with RET kinase.

In another aspect, the present disclosure provides a crystal form C of the compound represented by Formula I (hereinafter referred to as "crystal form C").

The crystal form C has an X-ray powder diffraction pattern with Cu-Kα radiation comprising characteristic peaks at 2θ of 6.9±0.2°, 8.6±0.2°, 11.5±0.2°, 13.9±0.2°, 16.9±0.2°, 17.9±0.2°, 18.4±0.2°, 20.3±0.2°, 23.8±0.2° and 26.9±0.2° in degrees.

Preferably, the crystal form C has an X-ray powder diffraction pattern with Cu-Kα radiation comprising characteristic peaks at 2θ of 6.9±0.2°, 8.6±0.2°, 9.3±0.2°, 9.7+0.2°, 11.5±0.2°, 13.9±0.2°, 14.9±0.2°, 15.8±0.2°, 16.3±0.2°, 16.9±0.2°, 17.9±0.2°, 18.4±0.2°, 20.3±0.2°, 23.2±0.2°, 23.8±0.2°, 24.4±0.2°, 25.3±0.2°, 26.9±0.2° and 28.2±0.2° in degrees.

More preferably, the X-ray powder diffraction pattern with Cu-Kα radiation of the crystal form C shows characteristic peaks at 2θ of the following positions in degrees and relative intensities, and the details are shown in Table 3.

**Table 3**

| number of peak | 2θ (°) | d (Å) | relative intensity (%) |
|---|---|---|---|
| 1 | 6.9 | 12.7 | 100 |
| 2 | 7.9 | 11.2 | 3.5 |
| 3 | 8.6 | 10.3 | 16.7 |
| 4 | 9.3 | 9.5 | 4.3 |
| 5 | 9.7 | 9.1 | 5.9 |
| 6 | 11.5 | 7.7 | 15.9 |
| 7 | 13.3 | 6.6 | 2.7 |
| 8 | 13.9 | 6.3 | 44.4 |
| 9 | 14.5 | 6.1 | 10.1 |
| 10 | 14.9 | 5.9 | 5 |
| 11 | 15.5 | 5.7 | 4.1 |
| 12 | 15.8 | 5.6 | 11.5 |
| 13 | 16.3 | 5.4 | 5.5 |
| 14 | 16.9 | 5.2 | 20.4 |
| 15 | 17.9 | 4.9 | 30.3 |
| 16 | 18.4 | 4.8 | 26 |
| 17 | 19.4 | 4.6 | 2.6 |
| 18 | 19.9 | 4.5 | 9.4 |
| 19 | 20.3 | 4.4 | 17.4 |
| 20 | 20.7 | 4.3 | 7.2 |
| 21 | 21.3 | 4.2 | 3.1 |
| 22 | 22.4 | 4.0 | 3.1 |
| 23 | 23.2 | 3.8 | 8.5 |
| 24 | 23.8 | 3.7 | 18.8 |
| 25 | 24.4 | 3.7 | 5.9 |
| 26 | 24.8 | 3.6 | 1.8 |
| 27 | 25.3 | 3.5 | 10.1 |
| 28 | 26.3 | 3.4 | 13.3 |
| 29 | 26.9 | 3.3 | 59.5 |
| 30 | 27.6 | 3.2 | 1.8 |
| 31 | 28.2 | 3.2 | 12.6 |
| 32 | 28.8 | 3.1 | 2.9 |
| 33 | 29.5 | 3.0 | 2.2 |
| 34 | 30.4 | 2.9 | 2.9 |
| 35 | 30.8 | 2.9 | 3.4 |

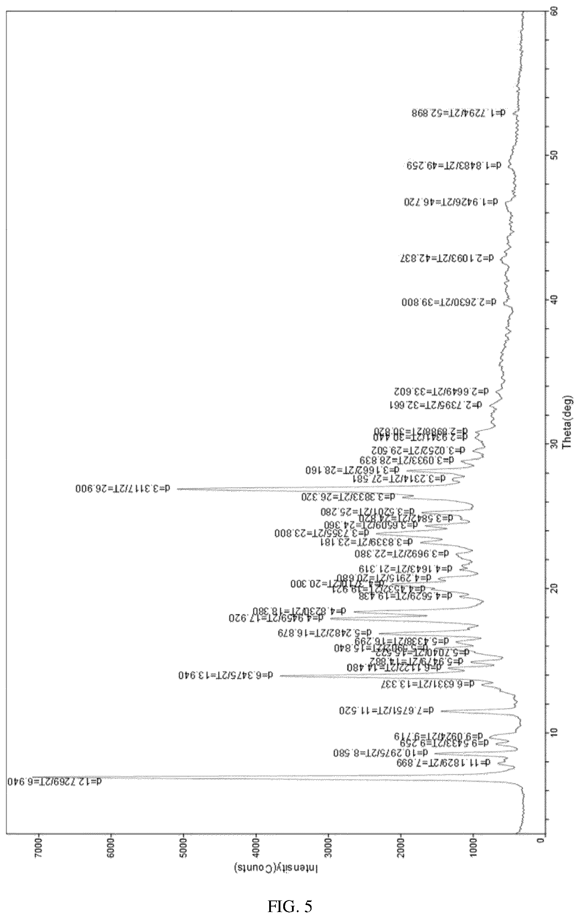

More preferably, the crystal form C has an X-ray powder diffraction pattern as substantially shown in FIG. 5.

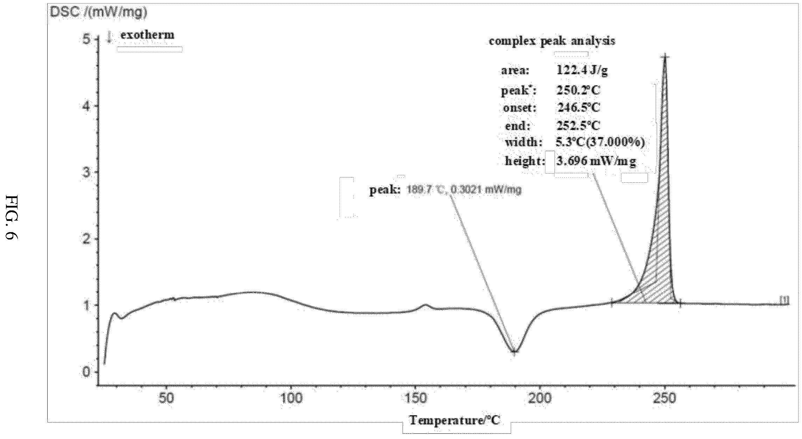

Further, the crystal form C has a DSC trace comprising a negative peak at 172°C to 195°C, an endothermic peak at 246°C to 252°C, and the DSC trace is substantially as shown in FIG. 6.

Correspondingly, the present disclosure provides a method for preparing the crystal form C, comprising:
adding an amorphous form of a compound represented by Formula I to a solvent to obtain a suspension, stirring the suspension at 5°C to 50°C for 1 to 7 days, separating a solid, and drying the solid under vacuum to obtain the crystal form C, and the solvent is selected from the group consisting of methyl acetate, ethyl acetate, isopropyl acetate, butyl acetate, isobutyl acetate, and a combination thereof.

Preferably, a weight-volume ratio of the compound represented by Formula I to the solvent is 1:10-1:50 in a unit of g/mL.

Preferably, the drying under vacuum is carried out at a temperature of 20°C to 60°C for 8 to 24 hours.

In another aspect, the present disclosure provides a pharmaceutical composition comprising a therapeutically effective amount of the crystal form C as an active ingredient. Preferably, in the pharmaceutical composition, the crystal form C can be mixed with one or more pharmaceutically acceptable solid or liquid diluents and/or excipients to prepare into a galenic preparation.

In a still another aspect, the present disclosure provides use of crystal form C or a pharmaceutical composition thereof in the manufacture of a medicament for treating a disease related to RET gene fusion or mutation, wherein the disease is preferably a cancer, and the cancer is preferably breast cancer, non-small cell lung cancer, or rectal cancer.

In a still another aspect, the present disclosure provides use of the crystal form C or a pharmaceutical composition thereof in the manufacture of a RET kinase inhibitor.

In addition, the present disclosure further provides a method for the treatment of a disease related to a RET gene fusion or mutation, and the method comprises administering a therapeutically effective amount of the crystal form C to a subject in need thereof, wherein the disease is preferably a cancer, wherein the cancer is preferably breast cancer, non-small cell lung cancer, or rectal cancer.

The present disclosure further provides a method for inhibiting RET kinase, and the method comprises contacting the crystal form C or a pharmaceutical composition thereof with RET kinase.

In another aspect, the present disclosure provides a crystal form D of the compound represented by Formula I (hereinafter referred to as "crystal form D").

The crystal form D has an X-ray powder diffraction pattern with Cu-Kα radiation comprising characteristic peaks at 2θ of 6.1±0.2°, 6.9±0.2°, 9.3±0.2°, 12.2±0.2°, 14.0±0.2°, 18.7±0.2° and 25.6±0.2° in degrees.

Preferably, the crystal form D has an X-ray powder diffraction pattern with Cu-Kα radiation comprising characteristic peaks at 2θ of 6.1±0.2°, 6.9±0.2°, 9.3±0.2°, 12.2±0.2°, 12.7±0.2°, 13.2±0.2°, 14.0±0.2°, 15.3±0.2°, 18.7±0.2°, 21.1±0.2°, 25.6±0.2° and 27.2±0.2° in degrees.

More preferably, the X-ray powder diffraction pattern with Cu-Kα radiation of the crystal form D shows characteristic peaks at 2θ of the following positions in degrees and relative intensities, and the details are shown in Table 4.

**Table 4**

| number of peak | 2θ (°) | d (Å) | relative intensity (%) |
|---|---|---|---|
| 1 | 6.1 | 14.4 | 9.3 |
| 2 | 6.9 | 12.8 | 100 |
| 3 | 9.3 | 9.5 | 5.7 |
| 4 | 12.2 | 7.2 | 7.3 |
| 5 | 12.7 | 7.0 | 1.4 |
| 6 | 13.2 | 6.7 | 1.3 |
| 7 | 14.0 | 6.3 | 31.7 |
| 8 | 15.3 | 5.8 | 3.9 |
| 9 | 15.7 | 5.6 | 4.8 |
| 10 | 18.7 | 4.8 | 17.3 |
| 11 | 21.1 | 4.2 | 2.4 |
| 12 | 21.5 | 4.1 | 2.5 |
| 13 | 25.6 | 3.5 | 5 |
| 14 | 27.2 | 3.3 | 1.8 |
| 15 | 28.5 | 3.1 | 1.3 |
| 16 | 31.1 | 2.9 | 2.6 |
| 17 | 32.6 | 2.7 | 2.6 |
| 18 | 35.7 | 2.5 | 1.2 |
| 19 | 38.0 | 2.4 | 1.2 |

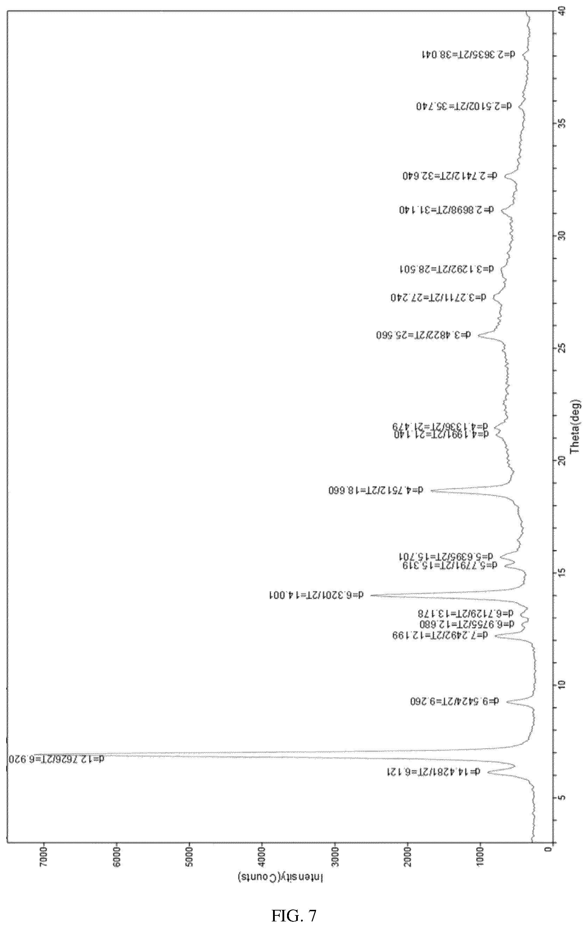

More preferably, the crystal form D has an X-ray powder diffraction pattern substantially as shown in FIG. 7.

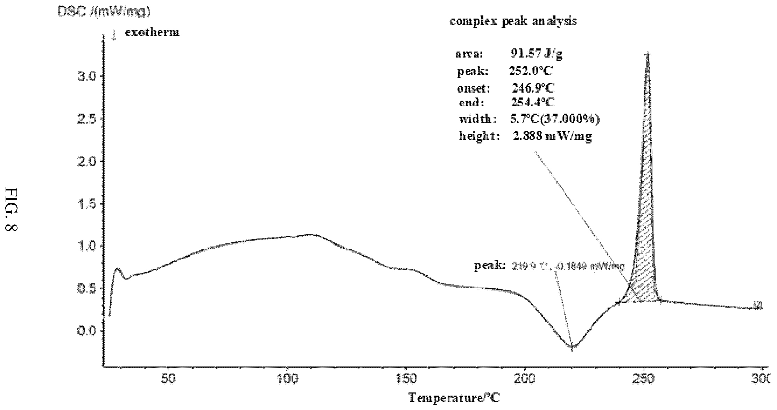

Further, the crystal form D has a DSC trace comprising a negative peak at 200°C to 235°C, an endothermic peak at 247°C to 254°C, and the DSC trace is substantially as shown in FIG. 8.

Correspondingly, the present disclosure provides a method for preparing the crystal form D, comprising:
adding an amorphous form of a compound represented by Formula I to N,N-dimethylacetamide to obtain a mixture, stirring the mixture at 10°C to 50°C for dissolution to obtain a clear solution, adding water for precipitation, separating a solid, and drying the solid under vacuum to obtain the crystal form D.

Preferably, a weight-volume ratio of the compound represented by Formula I to N,N-dimethylacetamide is 1:20-1:50 in a unit of g/mL.

Preferably, a volume ratio of N,N-dimethylacetamide to water is 1:1-1:4.

Preferably, the drying under vacuum is carried out at a temperature of 20°C to 60°C for 8 to 24 hours.

In another aspect, the present disclosure provides a pharmaceutical composition comprising a therapeutically effective amount of the crystal form D as an active ingredient. Preferably, in the pharmaceutical composition, the crystal form D can be mixed with one or more pharmaceutically acceptable solid or liquid diluents and/or excipients to prepare into a galenic preparation.

In a still another aspect, the present disclosure provides use of crystal form D or a pharmaceutical composition thereof in the manufacture of a medicament for treating a disease related to RET gene fusion or mutation, wherein the disease is preferably a cancer, and the cancer is preferably breast cancer, non-small cell lung cancer, or rectal cancer.

In a still another aspect, the present disclosure provides use of the crystal form D or a pharmaceutical composition thereof in the manufacture of a RET kinase inhibitor.

In addition, the present disclosure further provides a method for the treatment of a disease related to a RET gene fusion or mutation, and the method comprises administering a therapeutically effective amount of the crystal form D to a subject in need thereof, wherein the disease is preferably a cancer, wherein the cancer is preferably breast cancer, non-small cell lung cancer, or rectal cancer.

The present disclosure further provides a method for inhibiting RET kinase, and the method comprises contacting the crystal form D or a pharmaceutical composition thereof with RET kinase.

In another aspect, the present disclosure provides a crystal form E of the compound represented by Formula I (hereinafter referred to as "crystal form E").

The crystal form E has an X-ray powder diffraction pattern with Cu-Kα radiation comprising characteristic peaks at 2θ of 6.3±0.2°, 12.7±0.2°, 13.3±0.2°, 16.9±0.2°, 17.9±0.2°, 23.4±0.2° and 27.0±0.2° in degrees.

Preferably, the crystal form E has an X-ray powder diffraction pattern with Cu-Kα radiation comprising characteristic peaks at 2θ of 6.3±0.2°, 8.6±0.2°, 12.7±0.2°, 13.3±0.2°, 14.9±0.2°, 16.9±0.2°, 17.9±0.2°, 19.1±0.2°, 21.2±0.2°, 21.9±0.2°, 23.4±0.2°, 27.0±0.2° and 27.7±0.2° in degrees.

More preferably, the X-ray powder diffraction pattern with Cu-Kα radiation of the crystal form E shows characteristic peaks at 2θ of the following positions in degrees and relative intensities, and the details are shown in Table 5.

**Table 5**

| number of peak | 2θ (°) | d (Å) | relative intensity (%) |
|---|---|---|---|
| 1 | 6.3 | 14.0 | 100 |
| 2 | 8.6 | 10.2 | 2.9 |
| 3 | 9.6 | 9.2 | 0.8 |
| 4 | 12.7 | 7.0 | 26 |
| 5 | 13.3 | 6.6 | 11.8 |
| 6 | 14.9 | 5.9 | 3.7 |
| 7 | 16.6 | 5.3 | 4 |
| 8 | 16.9 | 5.2 | 6 |
| 9 | 17.9 | 4.9 | 9 |
| 10 | 19.1 | 4.6 | 9 |
| 11 | 21.2 | 4.2 | 3.5 |
| 12 | 21.9 | 4.1 | 1.6 |
| 13 | 23.4 | 3.8 | 7.6 |
| 14 | 27.0 | 3.3 | 16.9 |
| 15 | 27.7 | 3.2 | 4 |
| 16 | 29.9 | 3.0 | 1.5 |
| 17 | 30.7 | 2.9 | 0.8 |
| 18 | 32.7 | 2.7 | 0.5 |

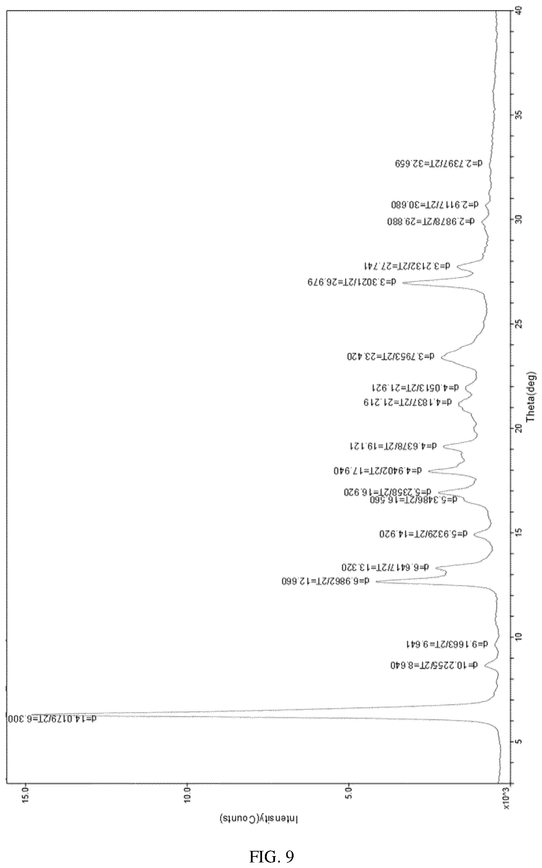

More preferably, the crystal form E has an X-ray powder diffraction pattern substantially as shown in FIG. 9.

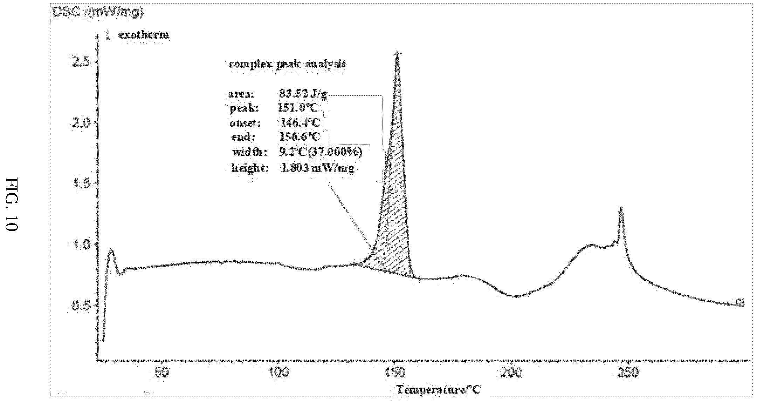

Further, the crystal form E has a DSC trace comprising an endothermic peak at 146°C to 156°C, and the DSC trace is substantially as shown in FIG. 10.

Correspondingly, the present disclosure provides a method for preparing the crystal form E, comprising:
adding a compound represented by Formula I to dimethyl sulfoxide to obtain a mixture, heating the mixture to 70°C to 80°C, stirring the mixture for 10-30 minutes to obtain a clear solution after dissolution, cooling to 0°C to 20°C for precipitation, separating a solid, and drying the solid under vacuum to obtain the crystal form E.

Preferably, a weight-volume ratio of the compound represented by Formula I to dimethyl sulfoxide is 1:5-1:10 in a unit of g/mL.

Preferably, the drying under vacuum is carried out at a temperature of 20°C to 60°C for 8 to 24 hours.

In another aspect, the present disclosure provides a pharmaceutical composition comprising a therapeutically effective amount of the crystal form E as an active ingredient. Preferably, in the pharmaceutical composition, the crystal form E can be mixed with one or more pharmaceutically acceptable solid or liquid diluents and/or excipients to prepare into a galenic preparation.

In a still another aspect, the present disclosure provides use of crystal form E or a pharmaceutical composition thereof in the manufacture of a medicament for treating a disease related to RET gene fusion or mutation, wherein the disease is preferably a cancer, and the cancer is preferably breast cancer, non-small cell lung cancer, or rectal cancer.

In a still another aspect, the present disclosure provides use of the crystal form E or a pharmaceutical composition thereof in the manufacture of a RET kinase inhibitor.

In addition, the present disclosure further provides a method for the treatment of a disease related to a RET gene fusion or mutation, and the method comprises administering a therapeutically effective amount of the crystal form E to a subject in need thereof, wherein the disease is preferably a cancer, wherein the cancer is preferably breast cancer, non-small cell lung cancer, or rectal cancer.

The present disclosure further provides a method for inhibiting RET kinase, and the method comprises contacting the crystal form E or a pharmaceutical composition thereof with RET kinase.

Compared with the prior art, the present disclosure offers the following beneficial effects.

The crystallization process of the crystal forms of the compound represented by Formula I of the present disclosure is simple, easy to operate, with little pollution, and can realize industrial production, and the crystal forms of the present disclosure has the advantages of high product purity, excellent physicochemical properties, good chemical and physical stability to high temperature/high humidity, excellent processing (filtration, drying, dissolution and tableting) adaptability, reproducibility, good dissolution, good dissolution time and biological release, small particle size, low hygroscopicity, and good fluidity, which is favorable to the medicament processing and the improvement of drugability, therefore the crystal forms have a good prospect of market application.

### BRIEF DESCRIPTION OF DRAWINGS

FIG. 1 is the X-ray powder diffraction pattern of the crystal form A prepared in Example 1.
FIG. 2 is the DSC trace of the crystal form A prepared in Example 1.
FIG. 3 is the X-ray powder diffraction pattern of the crystal form B prepared in Example 9.
FIG. 4 is the DSC trace of the crystal form B prepared in Example 9.
FIG. 5 is the X-ray powder diffraction pattern of the crystal form C prepared in Example 12.
FIG. 6 is the DSC trace of the crystal form C prepared in Example 12.
FIG. 7 is the X-ray powder diffraction pattern of the crystal form D prepared in Example 17.
FIG. 8 is the DSC trace of the crystal form D prepared in Example 17.
FIG. 9 is the X-ray powder diffraction pattern of the crystal form E prepared in Example 20.
FIG. 10 is the DSC trace of the crystal form E prepared in Example 20.
FIG. 11 is the X-ray powder diffraction pattern of the solid obtained in Preparation Example.

### DETAILED DESCRIPTION

### General definitions and terms

Unless otherwise specified, the technical and scientific terms used herein have the same meanings as commonly understood by those skilled in the art to which the present disclosure belongs. In case of conflict, the definition provided in this application will prevail. When expressing a certain amount, concentration, or other value or parameter in the form of a range, a preferred range, or a preferred upper limit and a preferred lower limit, it should be understood that it is equivalent to specifically disclosing any range by combining any pair of upper limit of the range or preferred value with any lower limit of the range or preferred value, regardless of whether the range is specifically disclosed. Unless otherwise specified, the numerical ranges listed herein are intended to include the endpoints of the range and all integers and fractions (decimals) within the range.

The terms "about" and "approximately" when used with a numerical variable usually mean that the value of the variable and all the values of the variable are within experimental error (for example, within the 95% confidence interval of the mean) or within ±10% of the specified value, or within a wider range.

The expression "comprise" or the synonymous similar expressions "include", "contain" and "have" are open-ended, and do not exclude additional unlisted elements, steps or components. The expression "consist of" excludes any unspecified elements, steps or components. The expression " essentially consist of" means that the scope is limited to the specified elements, steps or components, plus optional elements, steps or components that do not substantially affect the basic and new features of the claimed subject matter. It should be understood that the expression "comprise"/"comprising" encompasses the expressions "essentially consist of" and "consist of".

The term "optional" or "optionally" as used herein means that the event or situation subsequently described may or may not occur, and the expression includes the occurrence of the event or situation and the non-occurrence of the event or situation.

Unless otherwise specified, the percentages, parts, and the like used herein are all by weight.

As used herein, the term "crystal form" or "crystal" refers to any solid substance exhibiting a three-dimensional order, and producing a characteristic X-ray powder diffraction pattern with clearly-defined peaks as opposed to amorphous solid substances.

As used herein, the term "amorphous" refers to any solid substance that has no order in three dimensions.

As used herein, the term "X-ray powder diffraction (XRPD) pattern" refers to an experimentally observed diffraction pattern or a parameter, data or value derived therefrom. The XRPD pattern is usually characterized by peak position (abscissa) and/or peak intensity (ordinate).

As used herein, the term "2θ" refers to the peak position expressed in degrees (°) based on the setting in the X-ray diffraction experiment, and is usually the unit of abscissa in the diffraction pattern. If when the incident beam forms an angle θ with a certain lattice plane, the reflection is diffracted, the experimental setup needs to record the reflected beam at an angle of 2θ. It should be understood that the specific 2θ value of the specific crystal form mentioned herein is intended to mean the 2θ value (in degrees) measured using the X-ray diffraction experimental conditions described herein. For example, as described herein, Cu-Kα (Kα1 is 1.5418 Å) is used as the radiation source. The XRPD pattern herein can be collected, for example, on Rigaku MiniFlex-600 X-ray powder diffraction analyzer. Exemplary test conditions may be a scanning speed of 10°/min and a scanning step width of 0.01°.

As used herein, the term "substantially" for X-ray diffraction peaks means to take into account variations in the position and intensity of the representative peaks. For example, those skilled in the art will understand that the peak position (2θ) will show some changes, usually as much as 0.1-0.2 degrees (±0.1 to ±0.2 degrees), and the instrument used to measure diffraction will also cause some changes. In addition, those skilled in the art will understand that the relative peak intensity will vary due to differences between instruments, and the degree of crystallinity, preferred orientation, prepared sample surface, and other factors known to those skilled in the art, and should be regarded as merely a qualitative measurement.

As used herein, differential scanning calorimetry (DSC) measures the transition temperature when a crystal absorbs or releases heat due to a change in its crystal structure or melting of the crystal. For the same crystal form of the same compound, in continuous analysis, the error of thermal transition temperature and melting point is typically within about 5°C. When a compound is described as having a given DSC peak or melting point, it means that the DSC peak or melting point is ±5°C. "Substantially" also takes these temperature change into account. DSC provides an auxiliary method to distinguish different crystal forms. Different crystal forms can be identified according to their different transition temperature characteristics. It should be pointed out that for a mixture, its DSC peak or melting point may vary in a larger range. In addition, due to the decomposition of the substance in the process of melting, the melting temperature is related to the heating rate. DSC trace can be measured, for example, on an instrument such as model NETZSCH DSC214 Polyma. Exemplary test conditions include heating rate of 10°C/min and temperature range of 25°C to 300°C.

### Pharmaceutical composition and administration

In one embodiment, the present disclosure provides a pharmaceutical composition which comprises a crystal form of the compound represented by Formula I and one or more pharmaceutically acceptable carriers.

The term "pharmaceutically acceptable carrier" as used herein refers to a solid or liquid diluent, adjuvant, excipient or vehicle administered with the therapeutic agent, and it is suitable for contact with human and/or other animal tissues within the scope of reasonable medical judgment without excessive toxicity, irritation, allergic reaction, or other corresponding problems or complications compared with reasonable benefits/risks.

The pharmaceutically acceptable carriers that can be used in the pharmaceutical composition of the present disclosure include, but are not limited to, sterile liquids, such as water and oils, including those of petroleum, animal, vegetable, or synthetic origin, such as soybean oil, peanut oil, mineral oil, etc. When the pharmaceutical composition is administered intravenously, water is an exemplary carrier. It is also possible to use physiological saline and aqueous glucose and glycerol aqueous solutions as liquid carriers, especially for injections. Suitable pharmaceutical excipients include glucose, starch, lactose, gelatin, maltose, sucrose, chalk, silica gel, glyceryl monostearate, sodium stearate, talc, sodium chloride, glycerin, propylene glycol, water, ethanol and the like. The composition may also contain small amounts of wetting agents, emulsifiers or pH buffering agents as needed. Oral preparations may contain standard carriers such as pharmaceutical grades of mannitol, lactose, starch, magnesium stearate, cellulose, sodium saccharin, magnesium carbonate and the like. Examples of suitable pharmaceutically acceptable carriers are described in Remington's Pharmaceutical Sciences (1990).

The composition of the present disclosure can act systemically and/or locally. For this purpose, they can be administered through a suitable route, such as by injection, intraarterial, subcutaneous, intravenous, intraperitoneal, intramuscular or transdermal administration; or by oral, nasal, buccal, transmucosal, topical administration, or in the form of ophthalmic preparations or by inhalation.

For these administration routes, the composition of the present disclosure can be administered in a suitable dosage form. The dosage forms include, but are not limited to, tablets, pills, capsules, lozenges, hard lozenges, powders, sprays, creams, ointments, suppositories, gels, aqueous suspensions, injections, elixirs, and syrups.

The pharmaceutical composition of the present disclosure can be prepared by any method well known in the art, for example, by mixing, dissolving, granulating, sugar coating, milling, emulsifying, lyophilizing and the like. The term "therapeutically effective amount" as used herein refers to the amount of a compound that will relieve one or more symptoms of the condition being treated to a certain extent after being administered.

The dosage regimen can be adjusted to provide the best desired response. For example, a single bolus can be administered, several divided doses can be administered over time, or the dose can be proportionally reduced or increased as indicated by the urgent need for the treatment situation. It should be noted that the dose value may vary with the type and severity of the condition to be alleviated, and may include single or multiple doses. It should be further understood that for any particular individual, the specific dosing regimen should be adjusted over time according to the needs of the individual and the professional judgment of the person administering the composition or supervising the administration of the composition.

Unless otherwise specified, as used herein, the term "treating/treatment" means reversing, alleviating, or inhibiting the disorder or condition to which such terms are applied, or the progress of one or more symptoms of such disorder or condition, or preventing such disorder or condition or one or more symptoms of such disorder or condition.

"Individual" as used herein includes human or non-human animals. Exemplary human individuals include human individuals suffering from diseases such as those described herein (referred to as patients) or normal individuals. In the present disclosure, "non-human animals" include all vertebrates, such as non-mammals (e.g., amphibians, reptiles, birds) and mammals, such as non-human primates, livestock, and/or domesticated animals (e.g., dogs, cats, sheep, cows, pigs, etc.).

The following examples further illustrate the present disclosure, but it is necessary to point out that the following examples are only used for the description of the content of the present disclosure, and do not limit the scope of protection of the present disclosure, and the protection scope of the present disclosure is defined by the appended claims.

### Preparation and characterization of the crystal form of the compound represented by Formula I

### Preparation Example

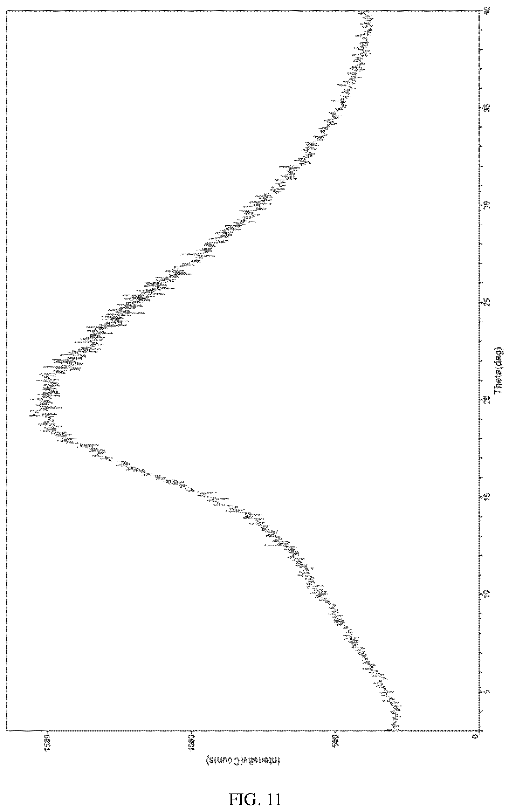

The compound represented by Formula I described in examples of the present disclosure was prepared according to the protocol in Example 2 of Patent WO2020/207419A1. That is, 2-(3,8-diazabicyclo[3.2.1]octan-3-yl)-6-methyl-N-(5-methyl-1H-pyrazol-3-yl)pyrimidin-4-amine hydrochloride (2c) (700 mg, 2.1 mmol), 6-(4-fluoro-1H-pyrazol-1-yl)pyridazine-3-formic acid (2g) (390 mg, 1.9 mmol) and PyBOP (1.63 g, 1.9 mmol) were dissolved in 20 mL of DMF, and cooled to 0 °C before DIPEA (810 mg, 6.3 mmol) was added. The reaction was kept at 0 °C for 30 min, quenched by adding 10 mL of water, and 150 mL of ethyl acetate was added. Washing with water was performed for three times (20 mLx3), followed by saturated brine once, dried over anhydrous sodium sulfate, and filtered. The solvent was removed under reduced pressure. After separation by silica gel column chromatography (the used eluents and volume ratio thereof: dichloromethane: methanol=20:1), the resulting crude product was further separated by silica gel preparative plate (the used developing agents and volume ratio thereof: dichloromethane: methanol=10:1) and concentrated under reduced pressure to obtain 400 mg of (6-(4-fluoro-1H-pyrazol-1-yl)pyridazin-3-yl)(3-(4-methyl-6-((5-methyl-1H-pyrazol-3-yl)amino)p yrimidin-2-yl)3,8-diazabicyclo[3.2.1]octane-8-yl)methanone (I-13), with a yield of 43%. The solid was amorphous as determined by XRPD assay and its X-ray powder diffraction pattern is shown in FIG. 11.

### Information of test instrument and test methods

X-ray powder diffraction instrument and test conditions involved in the present disclosure: the model of the X-ray diffraction instrument was Rigaku MiniFlex-600 Cu target; the operation method included a scanning speed of 10°/min and scanning step width of 0.01°.

DSC test conditions involved in the present disclosure: the model of the DSC detector was NETZSCH DSC214 Polyma; the operation method included a heating rate of 10°C/min and temperature range of 25°C to 300°C.

High-performance liquid chromatography (HPLC) test conditions involved in the present disclosure: the model of the liquid chromatograph was Agilent 1260; the chromatographic column was Welch XB C-18 250mm*4.6mm 3um; the detection wavelength was 260 nm; and the column temperature was 40°C.

### Example 1 Preparation of crystal form A

0.1 g of the compound represented by Formula I was mixed with 4 mL of ethanol to obtain a suspension, which was then stirred at 25°C for one day, and the solid was separated. The filter cake was then dried under vacuum at 50°C for 24 h to obtain 0.085 g of solid as crystal form A. Its X-ray powder diffraction pattern and DSC trace are shown in FIGs. 1-2, respectively.

### Example 2 Preparation of crystal form A

0.1 g of the compound represented by Formula I was mixed with 1 mL of methanol to obtain a suspension, which was then stirred at 5°C for 7 days, and the solid was separated. The filter cake was then dried under vacuum at 50°C for 24 h to obtain 0.088 g of solid as crystal form A. Its X-ray powder diffraction pattern and DSC trace are consistent with FIGs. 1-2, respectively.

### Example 3 Preparation of crystal form A

0.1 g of the compound represented by Formula I was mixed with 5 mL of n-propanol to obtain a suspension, which was then stirred at 50°C for one day, and the solid was separated. The filter cake was then dried under vacuum at 50°C for 24 h to obtain 0.082 g of solid as crystal form A. Its X-ray powder diffraction pattern and DSC trace are consistent with FIGs. 1-2, respectively.

### Example 4 Preparation of crystal form A

0.1 g of the compound represented by Formula I was mixed with 2 mL of isopropanol to obtain a suspension, which was then stirred at 25°C for 3 days, and the solid was separated. The filter cake was then dried under vacuum at 50°C for 24 h to obtain 0.086 g of solid as crystal form A. Its X-ray powder diffraction pattern and DSC trace are consistent with FIGs. 1-2, respectively.

### Example 5 Preparation of crystal form A

0.1g of the compound represented by Formula I was mixed with 2 mL of acetone to obtain a suspension, which was then stirred at 25°C for 3 days, and the solid was separated. The filter cake was then dried under vacuum at 50°C for 24 h to obtain 0.083 g of solid as crystal form A. Its X-ray powder diffraction pattern and DSC trace are consistent with FIGs. 1-2, respectively.

### Example 6 Preparation of crystal form A

g of the compound represented by Formula I was mixed with 2 mL of methyl isobutyl ketone to obtain a suspension, which was then stirred at 25°C for 3 days, and the solid was separated. The filter cake was then dried under vacuum at 50°C for 24 h to obtain 0.081 g of solid as crystal form A. Its X-ray powder diffraction pattern and DSC trace are consistent with FIGs. 1-2, respectively.

### Example 7 Preparation of crystal form A

0.1 g of the compound represented by Formula I was mixed with 2 mL of 2-butanone to obtain a suspension, which was then stirred at 25°C for three days, and the solid was separated. The filter cake was then dried under vacuum at 50°C for 24 h to obtain 0.082 g of solid as crystal form A. Its X-ray powder diffraction pattern and DSC trace are consistent with FIGs. 1-2, respectively.

### Example 8 Preparation of crystal form A

0.1 g of the compound represented by Formula I was mixed with 2 mL of ethanol and 2 mL of methanol to obtain a suspension, which was then stirred at 25°C for three days, and the solid was separated. The filter cake was then dried under vacuum at 50°C for 24 h to obtain 0.082 g of solid as crystal form A. The X-ray powder diffraction pattern and DSC trace are consistent with FIGs. 1-2, respectively.

### Example 9 Preparation of crystal form B

0.1 g of the compound represented by Formula I was mixed with 2 mL of dichloromethane to obtain a suspension, which was then stirred at 25°C for three days, and the solid was separated. The filter cake was then dried under vacuum at 50°C for 24 h to obtain 0.078 g of solid as crystal form B. Its X-ray powder diffraction pattern and DSC trace are shown in FIGs. 3-4, respectively.

### Example 10 Preparation of crystal form B

0.1 g of the compound represented by Formula I was mixed with 5 mL of dichloromethane to obtain a suspension, which was then stirred at 5°C for seven days, and the solid was separated. The filter cake was then dried under vacuum at 50°C for 24 h to obtain 0.075 g of solid as crystal form B. Its X-ray powder diffraction pattern and DSC trace are consistent with FIGs. 3-4, respectively.

### Example 11 Preparation of crystal form B

0.1 g of the compound represented by Formula I was mixed with 1 mL of dichloromethane to obtain a suspension, which was then stirred at 35°C for one day, and the solid was separated. The filter cake was then dried under vacuum at 50°C for 24 h to obtain 0.076 g of solid as crystal form B. Its X-ray powder diffraction pattern and DSC trace are consistent with FIGs. 3-4, respectively.

### Example 12 Preparation of crystal form C

0.1 g of the compound represented by Formula I was mixed with 2 mL of methyl acetate to obtain a suspension, which was then stirred at 25°C for three days, and the solid was separated. The filter cake was then dried under vacuum at 50°C for 24 h to obtain 0.082 g of solid as crystal form C. Its X-ray powder diffraction pattern and DSC trace are shown in FIGs. 5-6, respectively.

### Example 13 Preparation of crystal form C

0.1 g of the compound represented by Formula I was mixed with 1 mL of ethyl acetate to obtain a suspension, which was then stirred at 5°C for seven days, and the solid was separated. The filter cake was then dried under vacuum at 50°C for 24 h to obtain 0.083 g of solid as crystal form C. Its X-ray powder diffraction pattern and DSC trace are consistent with FIGs. 5-6, respectively.

### Example 14 Preparation of crystal form C

0.1 g of the compound represented by Formula I was mixed with 5 mL of isopropyl acetate to obtain a suspension, which was then stirred at 50°C for one day, and the solid was separated. The filter cake was then dried under vacuum at 50°C for 24 h to obtain 0.082 g of solid as crystal form C. Its X-ray powder diffraction pattern and DSC trace are consistent with FIGs. 5-6, respectively.

### Example 15 Preparation of crystal form C

0.1 g of the compound represented by Formula I was mixed with 2 mL of butyl acetate to obtain a suspension, which was then stirred at 25°C for three days, and the solid was separated. The filter cake was then dried under vacuum at 50°C for 24 h to obtain 0.081 g of solid as crystal form C. Its X-ray powder diffraction pattern and DSC trace are consistent with FIGs. 5-6, respectively.

### Example 16 Preparation of crystal form C

0.1 g of the compound represented by Formula I was mixed with 2 mL of isobutyl acetate to obtain a suspension, which was then stirred at 25°C for three days, and the solid was separated. The filter cake was then dried under vacuum at 50°C for 24 h to obtain 0.082 g of solid as crystal form C. Its X-ray powder diffraction pattern and DSC trace are consistent with FIGs. 5-6, respectively.

### Example 17 Preparation of crystal form D

0.1 g of the compound represented by Formula I was mixed with 2 mL of N,N-dimethylacetamide, stirred at 25°C to obtain a clear solution after dissolution, added with 2 mL of water for precipitation and the solid was separated. The filter cake was then dried under vacuum at 50°C for 24 h to obtain 0.078 g of solid as crystal form D. Its X-ray powder diffraction pattern and DSC trace are shown in FIGs. 7-8, respectively.

### Example 18 Preparation of crystal form D

0.1 g of the compound represented by Formula I was mixed with 2 mL of N,N-dimethylacetamide, stirred at 50°C to obtain a clear solution after dissolution, added with 5 mL of water for precipitation and the solid was separated. The filter cake was then dried under vacuum at 50°C for 24 h to obtain 0.082 g of solid as crystal form D. Its X-ray powder diffraction pattern and DSC trace are consistent with FIGs. 7-8, respectively.

### Example 19 Preparation of crystal form D

0.1 g of the compound represented by Formula I was mixed with 5 mL of N,N-dimethylacetamide, stirred at 10°C to obtain a clear solution after dissolution, added with 20 mL of water for precipitation and the solid was separated. The filter cake was then dried under vacuum at 50°C for 24 h to obtain 0.084 g of solid as crystal form D. Its X-ray powder diffraction pattern and DSC trace are consistent with FIGs. 7-8, respectively.

### Example 20 Preparation of crystal form E

0.1 g of the compound represented by Formula I was mixed with 0.5 mL of dimethyl sulfoxide, heated to 80°C, stirred for 30 minutes to obtain a clear solution after dissolution, and cooled to 20°C for precipitation, and the solid was separated. The filter cake was then dried under vacuum at 50°C for 24 h to obtain 0.061 g of solid as crystal form E. Its X-ray powder diffraction pattern and DSC trace are shown in FIGs. 9-10, respectively.

### Example 21 Preparation of crystal form E

0.1 g of the compound represented by Formula I was mixed with 1 mL of dimethyl sulfoxide, heated to 70°C, stirred for 10 minutes to obtain a clear solution after dissolution, and cooled to 0°C for precipitation, and the solid was separated. The filter cake was then dried under vacuum at 50°C for 24 h to obtain 0.049 g of solid as crystal form E. Its X-ray powder diffraction pattern and DSC trace are consistent with FIGs. 9-10, respectively.

### Preparation of amorphous form of compound represented by Formula I

The amorphous form of the compound represented by Formula I was prepared according to Example 2 of Patent WO2020/207419A1.

### Stability test

The amorphous form of compound represented by Formula I prepared in the described preparation examples, the crystal form A of the compound represented by Formula I prepared in Example 1, the crystal form B of the compound represented by Formula I prepared in Example 9, the crystal form C of the compound represented by Formula I prepared in Example 12, the crystal form D of the compound represented by Formula I prepared in Example 17, and the crystal form E of the compound represented by Formula I prepared in Example 20 were placed under various conditions: 75% RH, 92.5% RH, 40°C, 60°C, and illumination for 30 days, the X-ray powder diffraction pattern and purity of each crystal form were determined, and the specific results are shown in Table 6.

**Table 6**

| | crystal form A | crystal form B | crystal form C | crystal form D | crystal form E | amorphous form |
|---|---|---|---|---|---|---|
| day 0 | 99.85 | 99.80 | 99.75 | 99.60 | 99.76 | 96.77 |
| day 30 under 75% RH | 99.67 | 99.52 | 99.45 | 99.35 | 99.60 | 95.94 |
| day 30 under 92.5% RH | 99.48 | 99.23 | 99.08 | 99.01 | 99.50 | 95.23 |
| day 30 at 40°C | 99.80 | 99.68 | 99.51 | 99.42 | 99.70 | 96.17 (its XPRD pattern showed changes) |
| day 30 at 60°C | 99.77 | 99.62 | 99.38 | 99.33 | 99.65 | 95.98 (its XPRD pattern showed changes) |
| day 30 under illumination | 98.88 | 98.56 | 98.22 | 98.23 | 98.65 | 94.37 |

It can be seen from the results in Table 6 that crystal form A, crystal form B, crystal form C, crystal form D and crystal form E have better stability and higher purity, compared to the amorphous form.

### Granularity test

The amorphous form of the compound represented by Formula I prepared in Preparation Example, the crystal form A of the compound represented by Formula I prepared in Example 1, the crystal form B of the compound represented by Formula I prepared in Example 9, the crystal form C of the compound represented by Formula I prepared in Example 12, the crystal form D of the compound represented by Formula I prepared in Example 17, and the crystal form E of the compound represented by Formula I prepared in Example 20 were measured for granularity, and the specific results are shown in Table 7 below:

**Table 7**

| | amorphous form | crystal form A | crystal form B | crystal form C | crystal form D | crystal form E |
|---|---|---|---|---|---|---|
| D50 | 3.52 µm | 38.62 µm | 32.53 µm | 31.58 µm | 33.64 µm | 30.36 µm |
| D90 | 18.45 µm | 95.11 µm | 86.77 µm | 79.47 µm | 83.56 µm | 75.89 µm |

As can be seen from the results in Table 7, the granularity of the amorphous form was significantly smaller than that of crystal forms A, B, C, D and E (wherein D50 was 3.52 µm, which was not more than 12% of the crystal form D50 of the present disclosure, and D90 was 18.45 µm, which was not more than 25% of the crystal form D90 of the present disclosure), indicating that crystal forms provided by the present disclosure is more suitable for forming process of solid preparations (such as tablets, capsules or granules).

### Fluidity test

The amorphous form of the compound represented by Formula I prepared in Preparation Example, the crystal form A of the compound represented by Formula I prepared in Example 1, the crystal form B of the compound represented by Formula I prepared in Example 9, the crystal form C of the compound represented by Formula I prepared in Example 12, the crystal form D of the compound represented by Formula I prepared in Example 17, and the crystal form E of the compound represented by Formula I prepared in Example 20 were measured for angle of repose, and the specific results are shown in Table 8 below:

**Table 8**

| | amorphous form | crystal form A | crystal form B | crystal form C | crystal form D | crystal form E |
|---|---|---|---|---|---|---|
| 1 | 43.8° | 32.5° | 33.4° | 33.8° | 33.0° | 34.2° |
| 2 | 44.3° | 31.9° | 34.1° | 33.9° | 33.8° | 35.1° |
| 3 | 43.4° | 32.2° | 34.2° | 33.2.° | 33.3° | 34.8° |

The above results indicate that the angle of repose of the amorphous form was significantly larger than that of the crystal forms A, B, C, D and E. This indicates that the crystal forms A, B, C, D and E are significantly superior to the amorphous form in terms of fluidity and are more suitable for the forming process of solid preparations (such as tablets, capsules, or granules).

### Hygroscopicity test

The amorphous form of the compound represented by Formula I prepared in Preparation Example, the crystal form A of the compound represented by Formula I prepared in Example 1, the crystal form B of the compound represented by Formula I prepared in Example 9, the crystal form C of the compound represented by Formula I prepared in Example 12, the crystal form D of the compound represented by Formula I prepared in Example 17, and the crystal form E of the compound represented by Formula I prepared in Example 20 were placed in an environment of 75% RH and 25°C for 24 h and then each was weighed to calculate the hygroscopicity. The specific results are shown in Table 9 below.

**Table 9**

| | amorphous form | crystal form A | crystal form B | crystal form C | crystal form D | crystal form E |
|---|---|---|---|---|---|---|
| hygroscopicity | 0.45% | 0.02% | 0.05% | 0.06% | 0.08% | 0.05% |

The above results indicate that the hygroscopicity of the amorphous form is significantly greater than that of the crystal forms A, B, C, D and E, showing that the crystal forms A, B, C, D and E are significantly better than the amorphous form in terms of hygroscopicity.

Those skilled in the art can understand that the meaning or intended protection scope of the numerical value or numerical endpoint involved in the embodiments of the present disclosure is not limited to the number itself, and includes those allowable error ranges that have been widely accepted in the art, such as experimental error, measurement error, statistical error, random error, etc., and these error ranges are all included in the scope of the present disclosure.

It will be clear to those skilled in the art that many modifications and variations of the present disclosure can be made without departing from its spirit and scope. The specific embodiments described herein are provided by way of example only, and are not meant to be limited in any way. The true scope and spirit of the present disclosure are shown by the appended claims, and the description and examples are only exemplary.

## Claims

1. A crystal form A of a compound represented by Formula I, 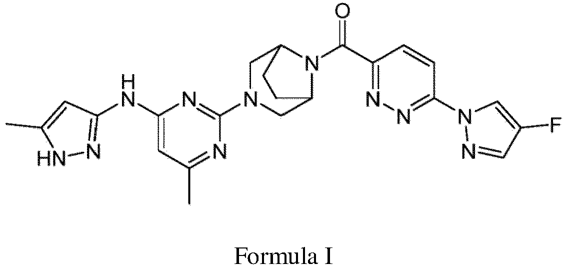 wherein the crystal form A has an X-ray powder diffraction pattern with Cu-Kα radiation comprising characteristic peaks at 2θ of 8.5±0.2°, 11.4±0.2°, 14.4±0.2°, 15.7±0.2°, 16.7±0.2°, 17.8±0.2°, 25.1±0.2° and 28.1±0.2° in degrees.

2. The crystal form A according to claim 1, wherein the crystal form A has an X-ray powder diffraction pattern with Cu-Kα radiation comprising characteristic peaks at 2θ of 7.0±0.2°, 8.5±0.2°, 11.4±0.2°, 13.1±0.2°, 14.4±0.2°, 15.7±0.2°, 16.7±0.2°, 17.8±0.2°, 20.2±0.2°, 21.2±0.2°, 22.0±0.2°, 23.1±0.2°, 24.3±0.2°, 25.1±0.2°, 26.2±0.2° and 28.1±0.2°in degrees.

3. The crystal form A according to claim 1 or 2, wherein the crystal form A has an X-ray powder diffraction pattern as shown in FIG. 1.

4. A method for preparing the crystal form A according to any one of claims 1 to 3, wherein the method comprises: adding an amorphous form of a compound represented by Formula I to a solvent to obtain a suspension, stirring the suspension at 5°C to 50°C for 1 to 7 days, separating a solid, and drying the solid under vacuum to obtain the crystal form A, and the solvent is selected from the group consisting of methanol, ethanol, n-propanol, isopropanol, acetone, methyl isobutyl ketone, 2-butanone, and a combination thereof.

5. The method according to claim 4, wherein a weight-volume ratio of the compound represented by Formula I to the solvent is 1:10-1:50 in a unit of g/mL.

6. A crystal form B of a compound represented by Formula I, 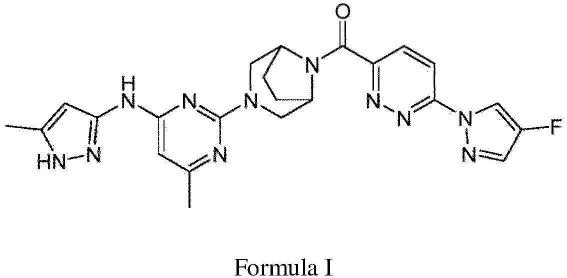 wherein the crystal form B has an X-ray powder diffraction pattern with Cu-Kα radiation comprising characteristic peaks at 2θ of 5.9±0.2°, 11.8±0.2°, 13.4±0.2°, 17.7±0.2°, 23.6±0.2° and 26.9±0.2° in degrees.

7. The crystal form B according to claim 6, wherein the crystal form B has an X-ray powder diffraction pattern with Cu-Kα radiation comprising characteristic peaks at 2θ of 5.9±0.2°, 9.0±0.2°, 9.9±0.2°, 11.8±0.2°, 13.4±0.2°, 17.7±0.2°, 21.9±0.2°, 23.6±0.2°, 24.5±0.2°, 26.9±0.2° and 29.9±0.2° in degrees.

8. The crystal form B according to claim 6 or 7, wherein the crystal form B has an X-ray powder diffraction pattern as shown in FIG. 3.

9. A method for preparing the crystal form B according to any one of claims 6 to 8, wherein the method comprises: adding an amorphous form of a compound represented by Formula I to dichloromethane to obtain a suspension, stirring the suspension at 5°C to 35°C for 1 to 7 days, separating a solid, and drying the solid under vacuum to obtain the crystal form B.

10. The method according to claim 9, wherein a weight-volume ratio of the compound represented by Formula I to dichloromethane is 1:10-1:50 in a unit of g/mL.

11. A crystal form C of a compound represented by Formula I, 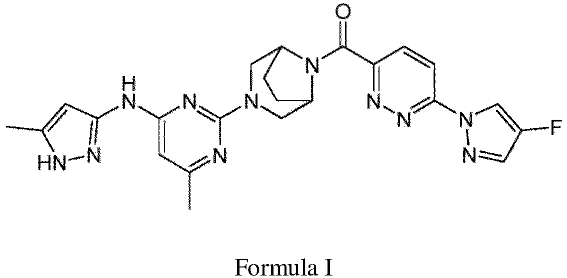 wherein the crystal form C has an X-ray powder diffraction pattern with Cu-Kα radiation comprising characteristic peaks at 2θ of 6.9±0.2°, 8.6±0.2°, 11.5±0.2°, 13.9±0.2°, 16.9±0.2°, 17.9±0.2°, 18.4±0.2°, 20.3±0.2°, 23.8±0.2° and 26.9±0.2° in degrees.

12. The crystal form C according to claim 11, wherein the crystal form C has an X-ray powder diffraction pattern with Cu-Kα radiation comprising characteristic peaks at 2θ of 6.9±0.2°, 8.6±0.2°, 9.3±0.2°, 9.7±0.2°, 11.5±0.2°, 13.9±0.2°, 14.9±0.2°, 15.8±0.2°, 16.3±0.2°, 16.9±0.2°, 17.9±0.2°, 18.4±0.2°, 20.3±0.2°, 23.2±0.2°, 23.8±0.2°, 24.4±0.2°, 25.3±0.2°, 26.9±0.2° and 28.2±0.2° in degrees.

13. The crystal form C according to claim 11 or 12, wherein the crystal form C has an X-ray powder diffraction pattern as shown in FIG. 5.

14. A method for preparing the crystal form C according to any one of claims 11 to 13, wherein the method comprises: adding an amorphous form of a compound represented by Formula I to a solvent to obtain a suspension, stirring the suspension at 5°C to 50°C for 1 to 7 days, separating a solid, and drying the solid under vacuum to obtain the crystal form C, and the solvent is selected from the group consisting of methyl acetate, ethyl acetate, isopropyl acetate, butyl acetate, isobutyl acetate, and a combination thereof.

15. The method according to claim 14, wherein a weight-volume ratio of the compound represented by Formula I to the solvent is 1:10-1:50 in a unit of g/mL.

16. A crystal form D of a compound represented by Formula I, 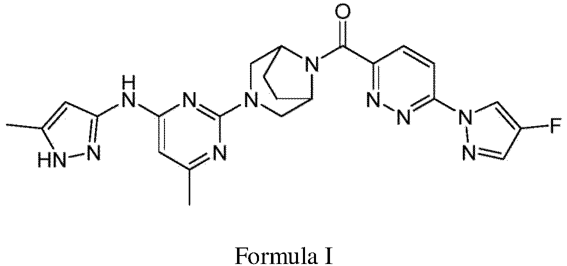 wherein the crystal form D has an X-ray powder diffraction pattern with Cu-Kα radiation comprising characteristic peaks at 2θ of 6.1±0.2°, 6.9±0.2°, 9.3±0.2°, 12.2±0.2°, 14.0±0.2°, 18.7±0.2° and 25.6±0.2° in degrees.

17. The crystal form D according to claim 16, wherein the crystal form D has an X-ray powder diffraction pattern with Cu-Kα radiation comprising characteristic peaks at 2θ of 6.1±0.2°, 6.9±0.2°, 9.3±0.2°, 12.2±0.2°, 12.7±0.2°, 13.2±0.2°, 14.0±0.2°, 15.3±0.2°, 18.7±0.2°, 21.1±0.2°, 25.6±0.2° and 27.2±0.2° in degrees.

18. The crystal form D according to claim 16 or 17, wherein the crystal form D has an X-ray powder diffraction pattern as shown in FIG. 7.

19. A method for preparing the crystal form D according to any one of claims 16 to 18, wherein the method comprises: adding an amorphous form of a compound represented by Formula I to N,N-dimethylacetamide to obtain a mixture, stirring the mixture at 10°C to 50°C for dissolution to obtain a clear solution, adding water for precipitation, separating a solid, and drying the solid under vacuum to obtain the crystal form D.

20. The method according to claim 19, wherein a weight-volume ratio of the compound represented by Formula I to N,N-dimethylacetamide is 1:20-1:50 in a unit of g/mL.

21. The method according to claim 19 or 20, wherein a volume ratio of N,N-dimethylacetamide to water is 1:1-1:4.

22. A crystal form E of a compound represented by Formula I, 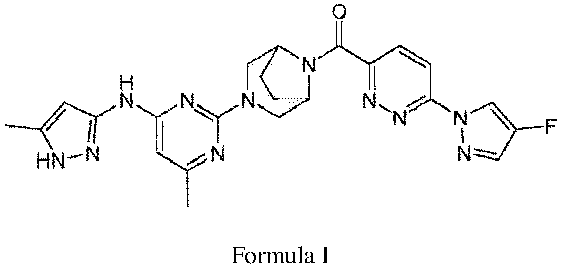 wherein the crystal form E has an X-ray powder diffraction pattern with Cu-Kα radiation comprising characteristic peaks at 2θ of 6.3±0.2°, 12.7±0.2°, 13.3±0.2°, 16.9±0.2°, 17.9±0.2°, 23.4±0.2° and 27.0±0.2° in degrees.

23. The crystal form E according to claim 22, wherein the crystal form E has an X-ray powder diffraction pattern with Cu-Kα radiation comprising characteristic peaks at 2θ of 6.3±0.2°, 8.6±0.2°, 12.7±0.2°, 13.3±0.2°, 14.9±0.2°, 16.9±0.2°, 17.9±0.2°, 19.1±0.2°, 21.2±0.2°, 21.9±0.2°, 23.4±0.2°, 27.0±0.2° and 27.7±0.2° in degrees.

24. The crystal form E according to claim 22 or 23, wherein the crystal form E has an X-ray powder diffraction pattern as shown in FIG. 9.

25. A method for preparing the crystal form E according to any one of claims 22 to 24, wherein the method comprises: adding an amorphous form of a compound represented by Formula I to dimethyl sulfoxide to obtain a mixture, heating the mixture to 70°C to 80°C, stirring the mixture for 10-30 minutes to obtain a clear solution after dissolution, cooling to 0°C to 20°C for precipitation, separating a solid, and drying the solid under vacuum to obtain the crystal form E.

26. The method according to claim 25, wherein a weight-volume ratio of the compound represented by Formula I to dimethyl sulfoxide is 1:5-1:10 in a unit of g/mL.

27. A pharmaceutical composition, comprising a therapeutically effective amount of the crystal form A according to any one of claims 1 to 3, the crystal form B according to any one of claims 6 to 8, the crystal form C according to any one of claims 11 to 13, the crystal form D according to any one of claims 16 to 18, or the crystal form E according to any one of claims 22 to 24, and a pharmaceutically acceptable excipient.

28. Use of the crystal form A according to any one of claims 1 to 3, the crystal form B according to any one of claims 6 to 8, the crystal form C according to any one of claims 11 to 13, the crystal form D according to any one of claims 16 to 18, the crystal form E according to any one of claims 22 to 24, or the pharmaceutical composition according to claim 27 in the manufacture of a medicament for treating a disease related to RET gene fusion or mutation, wherein the disease is preferably a cancer, and the cancer is preferably breast cancer, non-small cell lung cancer, or rectal cancer.

29. Use of the crystal form A according to any one of claims 1 to 3, the crystal form B according to any one of claims 6 to 8, the crystal form C according to any one of claims 11 to 13, the crystal form D according to any one of claims 16 to 18, the crystal form E according to any one of claims 22 to 24, or the pharmaceutical composition according to claim 27 in the manufacture of a RET kinase inhibitor.
